# EUROPEAN PATENT SPECIFICATION

(11) **EP 4 174 079 B1**
(45) Date of publication and mention of the grant of the patent: **24.04.2024**
(21) Application number: 22461625.0
(22) Date of filing: 21.10.2022
(51) Int. Cl.: C07F 15/00, A61P 35/00, A61K 31/282

(54) **CISPLATIN DERIVATIVE WITH UV / VIS LIGHT-CONTROLLED CYTOTOXICITY , METHOD OF PRODUCING THEREOF AND ITS USE IN ANTI-CANCER THERAPY**
CISPLATIN-DERIVAT MIT DURCH UV/VIS-LICHT KONTROLLIERTER ZYTOTOXIZITÄT, VERFAHREN ZU SEINER HERSTELLUNG UND VERWENDUNG IN DER KREBSTHERAPIE
DÉRIVÉ DE CISPLATINE À CYTOTOXICITÉ CONTRÔLÉE PAR LA LUMIÈRE UV/VIS, PROCÉDÉ DE SA PRODUCTION ET UTILISATION DANS LA THÉRAPIE ANTI-CANCÉREUSE

(30) Priority: 02.11.2021 PL 43940321
(43) Date of publication of application: 03.05.2023
(73) Proprietor: UNIWERSYTET JAGIELLONSKI, 31-007 Kraków (PL)
(72) Inventor: Szczubialka, Krzysztof, 32-442 Krzywaczka (PL); Stolarek, Marta, 30-392 Kraków (PL); Nowakowska, Maria, 30-433 Kraków (PL); Kaminski, Kamil, 30-733 Kraków (PL)
(74) Representative: Witek, Rafal

(56) References cited:
- SAKAI KEN ET AL: "Syntheses, antitumor activity, and molecular mechanics studies of cis-PtCl2(pzH)2 (pzH = pyrazole) and related complexes. Crystal structure of a novel Magnus-type double-salt [Pt(pzH)4][PtCl4][cis-PtCl2(pzH)2]2 involving two perpendicularly aligned 1D chains", INORGANICA CHIMICA ACTA, vol. 297, no. 1-2, 1 January 2000 (2000-01-01), pages 64-71, XP093026455,

## Description

The invention relates to a cisplatin derivative with UV/VIS (ultraviolet/visible) light-controlled cytotoxicity and a method of obtaining this derivative. The present invention provides a cisplatin derivative applicable in anti-cancer therapy.

The idea of using light for therapeutic purposes is known in the state of the art and has been used for more than a century. To date, the best-known example is photodynamic therapy (PDT), which is mainly used in the treatment of cancer [1]. PDT involves administration of a substance called photosensitiser, which, upon light absorption and electronic excitation, transfers the absorbed energy to oxygen molecules, forming reactive oxygen species (ROS), the actual active agents that destroys cancer cells. Photopharmacology is based on a different approach towords the therapeutic use of light [2]. It involves the use of a photoactive molecule, which is itself a drug, and can also be attached to a drug molecule or to a cellular structure such as ion channel. The pharmacological activity of such a system can be modified by light absorption followed by an irreversible photochemical event involving the molecule, such as photodissociation, intramolecular transfer of a chemical group, hydrogen or electron, or a reversible significant change in the shape of the molecule. Changing the molecule structure, and thus its size and physicochemical properties (dipole moment, solubility, stiffness), can alter its biological properties and pharmacological activity resulting in a change (enhancement or reduction) of the therapeutic effect. Molecules capable of reversibly changing their structure under the absorption of light of the appropriate wavelength are called photoswitches. The earliest and the most widely used type of photoswitches are azobenzene-based compounds that can undergo *trans-cis* (E/Z) photoisomerisation [3]. One of the photopharmacological strategies is to introduce a photoswitch molecule in place of a structurally similar part of the drug molecule. This results in an isostere of the drug containing the photoswitch. Such a procedure is called 'azologisation' because it was originally applied to azobenzene derivatives used as photoswitches. Azologisation can be applied to many drugs that are derivatives of stilbene, N-phenylbenzamide and other types of (hetero)aryl-(hetero)arylamides, benzyl anilines and benzyl phenyl (thio)ethers [2]. The first photopharmacological system, which involved activation of ion channels with light to control neuronal firing, was described in 2004 [4]. To date, photocontrol of antibiotics [5], enzymes [6], peptides and proteins [7], nucleic acids [8], oligonucleotides [9], ion channels (Cl, Ca, K, Na) [10] and cell receptors [11] has been investigated. Photopharmacological approaches have been proposed for the treatment of cancer [12], diabetes [13], hearing [14] and vision [15] pathologies, pain [16] and infectious diseases [17]. The photopharmacological concept in therapy is very attractive because light is in many respects an ideal therapeutic stimulus: visible (Vis) or infrared (IR) light is harmless to humans and can reach the affected area of the body in large quantity (intensity), with very good qualitative (wavelength), quantitative (exposure time) and spatial precision. Light can be delivered directly (skin, eyes), endoscopically (gastrointestinal tract, respiratory system, uterus, biliary tract, sinuses, urinary bladder), through the skin (testes, thyroid gland, some lymph nodes, muscles and bones) or through a skin incision (peritoneum, pancreas, liver, stomach, kidneys, prostate). The parts of the body most inaccessible to light are the brain and bone marrow, which can only be reached surgically. Light with a wavelength in the range limited to the infrared region (the so-called therapeutic window) is particularly useful and can penetrate into the tissues under the skin to a depth of up to approximately 2 cm. Cis-PtCl₂(3,5-dimethylpyrazole)₂ used as anti-tumour agent is disclosed by Sakai Ken et Al in Inorganica Chimica Acta, vol. 297, no. 1-2, (2000-01-01), pages 64-71.

Photoswitches are also known in the state of the art. Compounds modified with them should be characterised by therapeutic activity upon irradiation with one wavelength and should lose their activity upon irradiation with another one, with both optimally being within the therapeutic window. Moreover, both forms of the photoswitch molecule should be stable (i.e., they should not spontaneously change from one into the other via thermal isomerisation, as is the case for most *cis* isomers of azobenzene derivatives), and all or at least the vast majority of molecules should undergo photoisomerisation upon absorption of light of a given wavelength. In other words, photo-stationary states (PSS), established after sufficiently long exposure to a selected wavelength, should contain virtually pure one of both isomers. Another requirement, particularly important if the photoswitch is to operate in the intracellular environment, is resistance to biochemical processes that may inactivate it, such as hydrolysis or reduction by the reduced form of glutathione (GSH). The latter is a common problem associated with azobenzene-based photoswitches [18]. Furthermore, the synthesis and subsequent functionalisation of the photoswitch should be easy. Unfortunately, the perfect photoswitch has not yet been developed. For example, the *cis* isomers of azobenzene-based photoswitches often thermally isomerise to the *trans* form too quickly to be used therapeutically. This is particularly a problem with photoswitches that absorb infrared light and/or operate in an aqueous environment. Furthermore, it is difficult to simultaneously achieve a long thermal half-life of an isomer and a high content of this photoisomer in PSS [19]. Consequently, numerous studies are being conducted and rapid progress has been made on a molecule that could meet all the requirements for the perfect photoswitch. Arylazopyrazoles (AAPs) were first synthesised almost 30 years ago [20], but their photoswitching properties have only been studied since 2014 (Fuchter's group) [21]. It has been found that the characteristics of compounds based on the AAP moiety make them almost perfect photoswitches [2, 22-24]. The conformation of the cis isomer of AAP is inaccessible to azobenzenes and can be sterically modified by substitution in the pyrazole ring [21]. Based on DFT calculations, a large difference in dipole moments between AAP isomers is expected, e.g., for the benzimidazole derivative of arylazopyrazole the difference in dipole moments between *trans* and cis isomers is 3.30 D [25]. In particular, AAP-type photoswitches, which are pyrazolylazophenyl ether derivatives, have advantages relevant to biomedical applications, i.e., they exhibit near-quantitative *trans-cis* photoisomerisation and the reverse photoisomerisation in the near UV (365 nm) and visible light (532 nm), respectively [26]. For visible light at 400 nm, the *trans-cis* photoisomerisation is not quantitative, but still very efficient (80%), so both photoisomerisations can be achieved using visible light alone [26]. In the case of azobenzene derivatives, such a high conversion was only achieved in bridged azobenzenes (diazocines) [27]. Furthermore, steric repulsion within cis isomer of AAP is reduced compared to azobenzene, resulting in a long-lived *cis* isomer (τ_{1/2}≈months, so AAP can be considered a P-type photochrome). Furthermore, pyrazolylazophenyl ethers are resistant to side photoreactions and the reduction by GSH [18, 26], which is a prerequisite for their intracellular, long-lived activity *in vivo.* It is also important to note that the pyrazole group is present in a number of compounds that exhibit biological activity [28, 29], including anticancer [30], antiviral [31], anticonvulsant [32] activity, and inhibitors of cyclooxygenase (COX) [33] and nitrification [34].

Cisplatin (cis-diamminedichloroplatinum (II), CDDP) is known in the state of the art and was first synthesised in 1845 by Michele Peyrone and named Peyrone's salt [1]. Its potent anticancer activity was accidentally discovered in 1967 by Rosenberg [2]. In 1978, the compound was approved as an anticancer drug by the FDA, and a year later in the UK and other European countries. Since then, it has become one of the most widely used anticancer drugs, primarily in the treatment of testicular, ovarian, cervical, head and neck, bladder and lung cancers, in the treatment of melanomas and lymphomas. The global market for cisplatin is estimated to be worth approximately $2 billion, and one in two cancer patients is treated with cisplatin or a cisplatin analogue [3]. Cisplatin is administered intravenously and binds to plasma proteins in 65-98% within 24 hours after rapid infusion and in 100% after slow infusion [4]. It enters cells by passive diffusion across the cell membrane [5] or is actively transported by copper transporter protein (CTR1) [6]. Hence, cells with higher CTR1 expression are more sensitive to cisplatin [7]. The concentration of chloride anions Cl⁻ inside the cell is much lower than outside the cell, so the ligand in the form of the chloride anion Cl⁻ in cisplatin is replaced by water molecules and a monovalent, positively charged complex is formed that preferentially reacts with the N7 atom in guanine within genomic or mitochondrial DNA strands. The complex then reacts with a nitrogen atom of a nucleobase of another nucleotide (guanine or adenine [8]), mainly in the same strand to form a cyclic adduct [9]. The adduct formation blocks the production of DNA, mRNA and proteins, ultimately leading to cell necrosis or apoptosis. Interestingly, *trans*-platinum compounds do not exhibit anticancer activity due to their inability to crosslink DNA and their rapid inactivation by thiols [10]. Furthermore, cisplatin induces oxidative stress through the formation of reactive oxygen species, such as hydroxyl radical or superoxide anion radical. Unfortunately, cisplatin does not react with DNA only. A second target is the thiol group of glutathione (GSH). It also reacts with the cytoskeleton, RNA and proteins containing thiol groups, such as metallothioneins [11]. These interactions are responsible for the severe adverse effects of cisplatin, which include nausea, vomiting, nephrotoxicity, ototoxicity, hepatotoxicity and neurotoxicity. Another challenge associated with the use of cisplatin is the observed sometimes resistance to the drug. Some cancers are inherently resistant to cisplatin (e.g., colon cancer and non-small cell lung cancer), while other develop resistance over time (e.g., ovarian cancer or small cell lung cancer). The development of resistance is due to reduced accumulation of the drug inside the cells, inactivation of GSH and metallothioneins and accelerated DNA repair [12]. Due to the clinical limitations of cisplatin, a large number of its derivatives have been synthesised and tested. Carboplatin and oxaliplatin are two examples that have been successfully introduced into clinical treatment. To be approved for clinical use, a platinum analogue must show at least one clinical advantage over cisplatin, such as activity against intrinsically cisplatin-resistant tumours, reduced side effects or the possibility of oral administration [13]. Structure-activity relationship (SAR) studies have shown that in order to exhibit anticancer activity, a new platinum complex should meet the following criteria [14]: the compound should be neutral, the compound must have two leaving groups that are in a cis position relative to each other and, consequently, both amino groups should also be in a mutual cis position, the leaving groups should have a moderate tendency to dissociate. Compounds with ligands that dissociate too readily are unacceptably toxic, whereas compounds with ligands bound too strongly are inactive. The platinum cation forms permanent coordination bonds with the nitrogen atoms of nitrogen-containing aromatic compounds [15]. Therefore, a number of cisplatin analogues with amino groups replaced by N-heterocyclic compounds have been synthesised and studied. Such cisplatin derivatives include picoplatin, in which the replacement of one amino group by ortho-picoline (i.e., 2-methylpyridine) resulted in a lower activity against cisplatin-sensitive tumours, but a higher activity against ovarian cancer and small cell lung cancer [16]. Examples of other N-heterocyclic rings used as ligands include moieties such as pyridine, 7-azaindol, rutaecarpine, imidazole, oxadiazole, oxoisoaporphine, benzothiazole, phenanthridine, 1,8-naphthyridine and 1,10-phenanthroline. Of particular interest are complexes of pyrazole and Pt²⁺-based ligands. A cisplatin analogue containing pyrazole (cis-PtCl₂(pzH)₂) was first synthesised by van Kralingen and co-workers [17]. Later, Sakai with a team proposed a modified synthetic method [18]. Cis-PtCl₂(pzH)₂ was found to have antitumour activity significantly lower than cisplatin [18, 19], and its 3,5-pyrazolodicarboxylic acid analogue was completely ineffective against a human gastric adenocarcinoma cell line (AGS) [18]. On the other hand, cationic multi-nuclear platinum complexes linked to 4,4'-dipyrazolylmethane formed bonds with various DNA strands and showed activity in mouse leukaemia cell line L1210 and in human ovarian cancer [20].

Given the current state of the art, a number of problems remain unresolved, such as: high toxicity of cisplatin and its derivatives used in anticancer therapy with respect to healthy cells, no possibility of manipulating the level of cytotoxicity of cisplatin or its derivatives with UV or VIS light, no possibility of cyclic converting cisplatin or its derivatives into a form with high or low toxicity, large number of side effects associated with current therapy with cisplatin and its derivatives leading to serious consequences such as infections, bleeding, exhaustion, vomiting, loss of appetite, diarrhoea, hearing loss, renal failure, difficulty breathing and secondary tumours, low efficacy of state-of-the-art cisplatin treatment emerging over time due to resistance development, including cross-resistance. Consequently, there is a need to develop and synthesise new cisplatin derivatives as potential anticancer drugs that could represent a revolutionised approach in the treatment of these conditions.

Unexpectedly, most of the technical problems indicated above can be solved in the presented invention.

The subject of the invention is a novel cisplatin derivative and its applications, which are defined in the appended claims.

During development of the present invention, a cisplatin derivative was obtained that was substituted with an arylazopyrazole moiety capable of reversible photoisomerisation under 360-400 nm (*trans-cis*) and 530 nm *(cis-trans)* light. AAP was attached to the platinum atom via a nitrogen atom of pyrazole. The resulting cisplatin derivative in the non-irradiated form (i.e., obtained directly in the synthesis) was shown to have relatively high toxicity towards cancer cells (mouse B16-F10 melanoma cells and mouse 4T1 breast cancer cells). Upon irradiation with 360-400 nm light, the compound is transformed into a much less toxic form In turn, that less toxic form is slowly transformed into the more toxic form without light involvement. The transformation from the less toxic form (obtained by irradiation at 360-400 nm) back to the more toxic form can be accelerated by irradiation with visible light at about 530 nm. The transformation of the compound from a less to more toxic form or vice versa by irradiation with light of the appropriate wavelengths can be performed repeatedly.

The photosensitivity of this compound allows temporal and spatial control of the potency of its anticancer effect, e.g., enhancing its action (increasing cytotoxicity) selectively in tumour tissue by irradiating the tumour with light at a wavelength of about 530 nm following administration of the compound in its less toxic form. By suitably modifying the chemical structure of the photosensitive ligands, it will be possible to modify the photochemical properties (including the wavelength of light used for irradiation, thermal stability of the less toxic form) and pharmacological properties (cytotoxicity, binding to proteins, etc.) of the photosensitive cisplatin derivatives of the invention.

The cisplatin derivative which is the subject of the invention has many advantages. A particular advantage is the ability to manipulate the level of cytotoxicity of this cisplatin derivative through its irradiation or lack thereof, its photochemical properties and the possibility of cyclically converting this cisplatin derivative into a form with high or low toxicity. Owing to this property, it is possible to purposely reduce systemic cytotoxicity by converting the drug into its active form only once it has reached the tumour tissue or only within that tissue. A further advantage of the subject invention is the increased selective anti-tumour effect, so that its use should be associated with fewer and less intense therapy-related side effects.

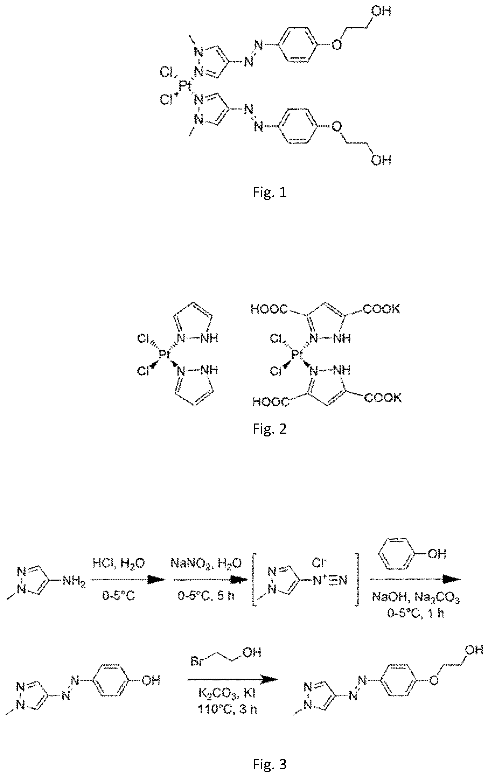

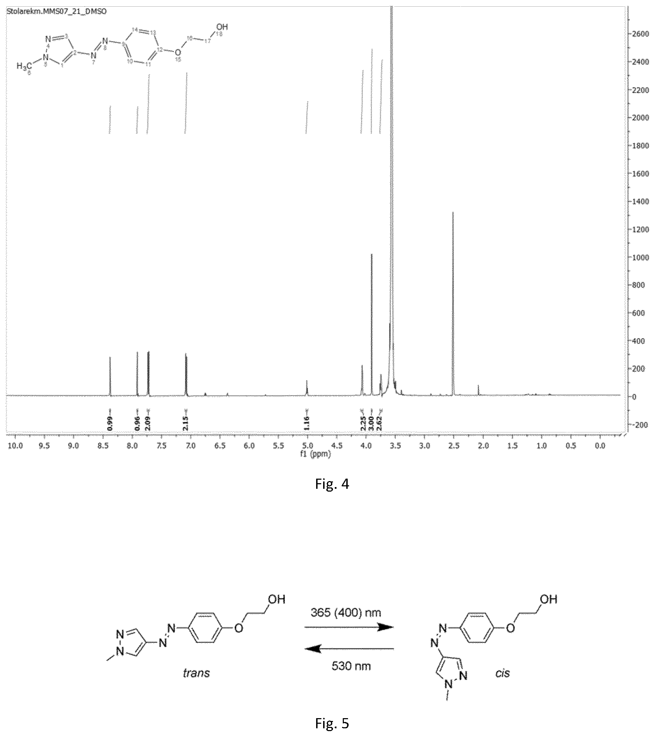

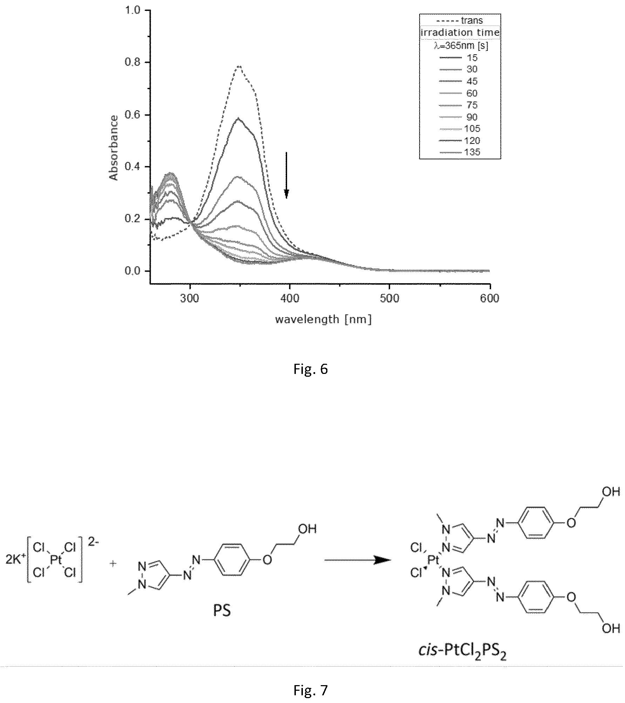

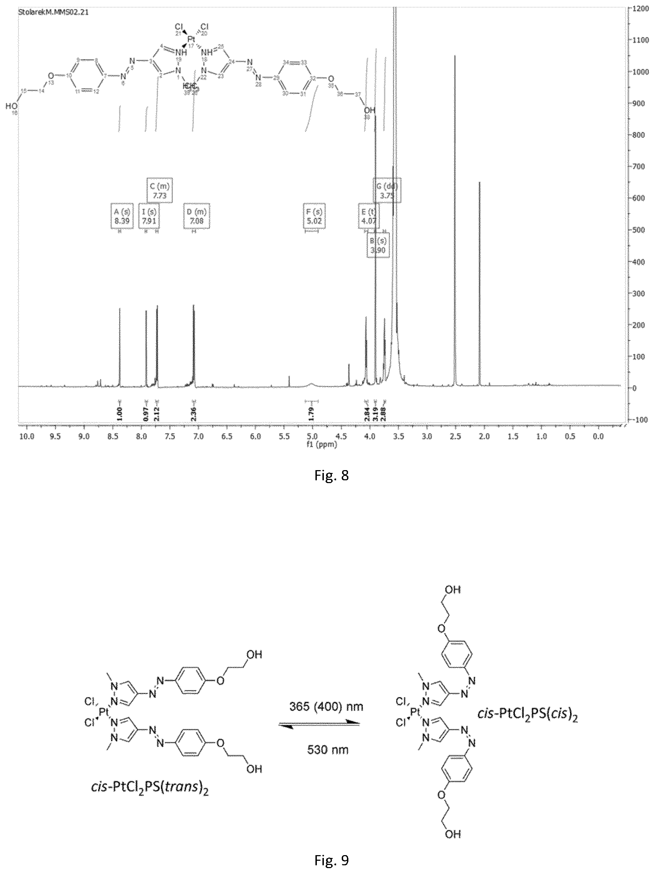

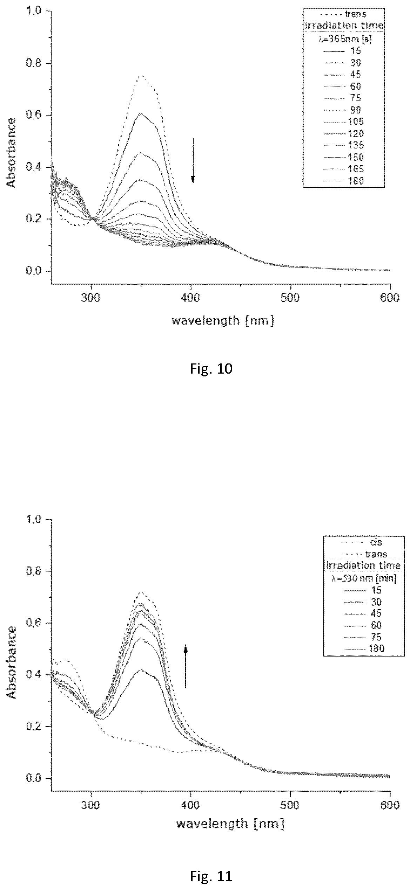

The subject of the invention in embodiments is illustrated in the drawings, in which Fig. 1 shows the structure of the novel cisplatin derivative (cis-PtCl₂PS₂), Fig. 2 shows the structures of cisplatin analogues known from the state of the art, with pyrazole and 3,5-dicarboxypyrazole as ligands [18], Fig. 3 shows a synthesis diagram for the photoswitch, Fig. 4 shows the NMR spectrum of the photoswitch, Fig. 5 shows the *cis-trans* photoisomerisation of the photoswitch, Fig. 6 shows the UV-Vis spectra of the photoswitch in its *trans* form (c=0.0125 mg/mL in DMSO) exposed to 365 nm light (xenon lamp), Fig. 7 shows a synthesis diagram for the novel cisplatin derivative, Fig. 8 shows the NMR spectrum of the novel cisplatin derivative, Fig. 9 shows the *cis-trans* photoisomerisation of the novel cisplatin derivative, Fig. 10 shows the UV/Vis spectra of the novel cisplatin derivative with the photoswitch in its *trans* form (c=0.0125 mg/mL in DMSO) exposed to 365 nm light (xenon lamp), Fig. 11 shows the UV/Vis spectra of the novel cisplatin derivative with the photoswitch in its *cis* form (c=0.015 mg/mL in DMSO) exposed to 530 nm light (530 nm LED, ThorLabs), Fig. 12 shows the viability of mouse 4T1 breast cancer cells after adding different concentrations of *cis*-PtCl₂PS₂ in DMSO with the photoswitch ligand in its *trans* and *cis* forms, Fig. 13 shows the proliferation of mouse B16-F10 melanoma cells after adding cis-PtCl₂PS₂ in DMSO (final concentration 1.06·10⁻⁵ M, 8.04 µg/mL) with the photoswitch ligand in its *trans* and *cis* forms, 46 h after administration of the compound in DMSO.

### Example 1

The photoswitch (PS) was obtained by a two-step synthesis route.

### Step 1

Step 1 followed a procedure described in the literature [5]. All reagents used in the photoswitch synthesis are commercially available. 4-amino-1-methylpyrazole (2.91 g, 30 mmol, 1 equivalent) was dissolved in 60 mL of water, followed by adding 14 mL of HCl (12.2 mol/L, 170 mmol, 5.67 equivalents). After cooling the solution to 0-5°C, a solution of NaNO₂ (2.7 g, 39 mmol, 1.3 equivalents) dissolved in 60 mL of water and pre-cooled to 0-5°C was slowly added. After cooling the mixture for 30 min in a 0°C bath, a solution of phenol (3.38 g, 36 mmol, 1.2 equivalents) and NaOH (3.2 g, 80 mmol) dissolved in 80 mL of water and pre-cooled to 0-5°C was slowly added. Then, a solution of Na₂CO₃ (10.6 g, 100 mmol) dissolved in 100 mL of water and pre-cooled to 0-5°C was slowly added. During this step, yellow-brown particles were formed and the reaction mixture was stirred for 1 hour while maintaining the temperature at 0-5°C. The mixture was then brought to pH~7 and the resulting suspension was filtered and washed on a Büchner funnel with 50 mL of distilled water. The filter cake was dried under vacuum at 25°C for 24 h to give the product as a yellow solid, pzAzophenol ((E)-4-((1-methyl-1H-pyrazol-4-yl)diazenyl)phenol) (5.71 g, yield: 94.2%).

### Step 2

The pzAzophenol ((E)-4-((1-methyl-1H-pyrazol-4-yl)diazenyl)phenol) obtained in Step 1 (1.00 g, 4.9 mmol, 1 equivalent) and K₂CO₃ (2.73 g, 19.8 mmol, 4 equivalents) were dissolved in 9 mL of DMF, and then KI (41 mg, 0.20 mmol, 0.05 equivalents) was added. 1-Bromo-2-ethanol (0.95 mL, 13.4 mmol, 2.7 equivalents) was added dropwise into the resulting mixture, followed by stirring at 110°C under a reflux condenser for 3 hours. The reaction mixture was quenched by adding water and stirred until a solid crystallized. The solid product obtained was filtered and washed with water. The filter cake was dissolved in 80 mL of ethyl acetate, after which the solution was dried over Na₂SO₄ and concentrated under reduced pressure. Purification was carried out by column chromatography with n-hexane:ethyl acetate 1:1 solvent. 920 mg of PS (((E)-2-(4-((1-methyl-1H-pyrazol-4-yl)diazenyl)phenoxy)ethan-1-ol)) was obtained (yield: 75%).

¹H NMR (600 MHz, DMSO) δ 8.38 (s, J = 8.7 Hz, 1H), 7.91 (d, J = 0.6 Hz, 1H), 7.72 (d, 2H), 7.08 (d, 2H), 5.01 (t, J = 5.5 Hz, 1H), 4.07 (t, J = 6.1, 3.6 Hz, 2H), 3.90 (s, 3H), 3.76-3.72 (m, 2H). The NMR spectrum confirms the assumed structure of the photoswitch.

Photoisomerisation of the above-obtained photoswitch was carried out and it was found that the synthesised photoswitch undergoes reversible photoisomerisation under irradiation with 365-400 nm light (*trans-cis* photoisomerisation) and 530 nm light (*cis-trans* photoisomerisation).

### Example 2

The synthesis of the cis-PtCl₂PS₂ complex was carried out according to a modified literature procedure. 100 mg (0.241 mmol, 1 equivalent) of K₂PtCl₄ was dissolved in 10 ml of distilled water, then 118 mg (0.48 mmol) of the photoswitch synthesised in Example 1 was suspended in 33 ml of ethanol, added to the aqueous solution of K₂PtCl₄ and stirred at room temperature for 2 hours. Then, 5 ml of ethanol was added to completely dissolve the photoswitch, after which the solution was stirred for further 46 hours at room temperature. The ethanol was removed under reduced pressure. The precipitated product was centrifuged off the remaining water (2 min, 10,000 rpm) and the supernatant decanted. 5 ml of diethyl ether was added, after which the suspension was stirred and the supernatant removed. The procedure of washing with diethyl ether was carried out three times. The product was dried at 22°C for 20 h in a vacuum oven. A cis-PtCl₂PS₂ complex was obtained in 77 mg (42%) yield.

Elemental analysis of the cis-PtCl₂PS₂ complex:
Experimental: C - 38.25 %; H - 3.98 %; N - 14.34 %.
Theoretical: (C₂₄H₂₈N₈O₄Cl₂Pt, MW 758.52): C - 38.00 %; H - 3.72 %; N - 14.77 %.
¹H NMR (600 MHz, DMSO) δ 8.39 (s, 2H), 7.91 (s, J = 8.4 Hz, 2H), 7.75-7.71 (m, 4H), 7.11-7.05 (m, 4H), 5.02 (s, 2H), 4.07 (t, 4H), 3.90 (s, J = 10.6 Hz, 6H), 3.75 (dd, J = 9.7, 4.9 Hz, 4H).

Elemental analysis and ¹H NMR spectrum confirm the structure of the cis-PtCl₂PS₂ complex.

The photoisomerisation of the cis-PtCl₂PS₂ complex can be followed by measuring UV-Vis spectra.

### Example 3

Cytotoxicity studies of the resulting photocomplex were performed on the 4T1 cell line (mouse breast cancer). 4T1 cancer cells were cultured on Petri dishes in a culture medium (DMEM high glucose) supplemented with 10% (vol/vol) fetal bovine serum FBS, at 37°C in a humidified atmosphere containing 5% (vol/vol) of CO₂. The cells were then seeded in 24-well plates and cultured for 5 hours. After this time, the cells were treated with 10 µl of the cis-PtCl₂PS₂ complex in its *cis* and *trans* forms in DMSO at 6 different concentrations: 1.06·10⁻⁵; 7.04·10⁻⁶; 4.69·10⁻⁶; 3.13·10⁻⁶; 2.09·10⁻⁶; 1.39·10⁻⁶ M and incubated for 24 h, 48 h and 72 h. 10 µl of DMSO were added to control wells. Crystal violet (CrV) assay was used to assess cell viability. After incubation, the medium was removed and the cells were washed twice with 1 ml of PBS, then 1 ml of 4% vol/vol formaldehyde/PBS mixture was added and incubated for 10 minutes, washed again with 1 ml of PBS and treated for 2 minutes with CrV solution. The unbound CrV was then removed by washing with water. After drying, a decolourisation solution was added to each well and incubated for 20 minutes. Finally, the absorbance of the resulting solution was measured at 540 nm, which was proportional to the number of viable cells.

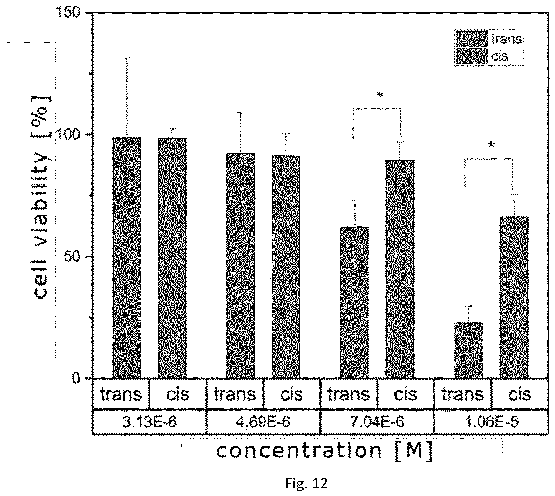

For the cis-PtCl₂PS₂ complex, cell-based studies showed significantly higher cytotoxicity of the cis-PtCl₂(*trans*-PS)₂ form than that of the cis-PtCl₂(*cis*-PS)₂ form after 46 h at a concentration of 7.0 µM or higher to mouse breast cancer cells (4T1) (Fig. 12). The difference in cell viability for both photoisomers of the complex increased over time, despite the fact that the less toxic photoisomer cis-PtCl₂(*cis*-PS)₂ gradually underwent thermal conversion to the more toxic cis-PtCl₂(*trans*-PS)₂.

### Example 4

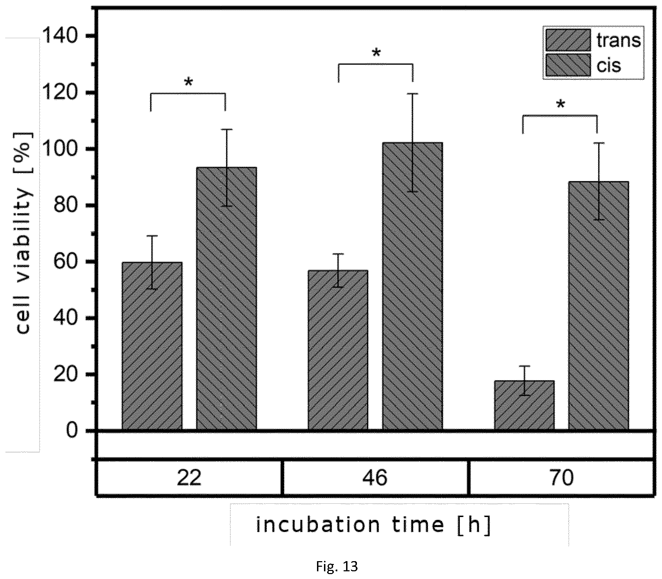

The toxicity of platinum(II) photocomplex was investigated using mouse B16-F10 melanoma cell line. B16-F10 cancer cells were cultured on Petri dishes in a culture medium (DMEM high glucose) supplemented with 10% (vol/vol) fetal bovine serum FBS, at 37°C in a humidified atmosphere containing 5% (vol/vol) of CO₂. The cells were then seeded in 24-well plates and cultured for 5 hours. After this time, the cells were treated with 10 µl of the cis-PtCl₂PS₂ complex in its *cis* and *trans* forms in DMSO at 6 different concentrations: 1.06·10⁻⁵; 7.04·10⁻⁶; 4.69·10⁻⁶; 3.13·10⁻⁶; 2.09·10⁻⁶; 1.39·10⁻⁶ M and incubated for 24 h, 48 h and 72 h. 10 µl of DMSO were added to control wells. Crystal violet (CrV) assay was used to assess cell viability. After incubation, the medium was removed and the cells were washed twice with 1 ml of PBS, then 1 ml of 4% vol/vol formaldehyde/PBS mixture was added and incubated for 10 minutes, washed again with 1 ml of PBS and treated for 2 minutes with CrV solution. The unbound CrV was then removed by washing with water. After drying, a decolourisation solution was added to each well and incubated for 20 minutes. Finally, the absorbance of the resulting solution was measured at 540 nm, which was proportional to the number of viable cells. For the cis-PtCl₂PS₂ complex, cell-based studies showed significantly higher cytotoxicity of the cis-PtCl₂(*trans*-PS)₂ form than that of the cis-PtCl₂(*cis*-PS)₂ form after 46 h at a concentration of 1.06·10⁻⁵ M to mouse melanoma cells (B16-F10) (Fig. 13). Importantly, the difference in cell viability for both photoisomers of the complex increased over time, despite the fact that the less toxic photoisomer cis-PtCl₂(*cis*-PS)₂ gradually underwent thermal conversion to the more toxic cis-PtCl₂(*trans*-PS)₂.

*In vitro* studies indicate that, unexpectedly, the cis-PtCl₂(*trans*-PS)₂ form of the complex is significantly more toxic than the cis-PtCl₂(*cis*-PS)₂ form of the complex. This means that it will become possible to locally increase the toxicity of the complex administered in the cis-PtCl₂(*cis*-PS)₂ form by irradiation with 530 nm light, resulting in conversion to the more toxic cis-PtCl₂(*trans*-PS)₂ form, or to locally reduce the toxicity of the complex administered in the cis-PtCl₂(*trans*-PS)₂ form by irradiation with 400 nm light, resulting in its conversion to the less toxic cis-PtCl₂(*cis*-PS)₂ form.

### Literature

1. X. Shi, C. Y. Zhang, J. Gao, Z. Wang, Recent advances in photodynamic therapy for cancer and infectious diseases. Wiley Interdiscip. Rev. Nanomedicine Nanobiotechnology. 11, 1-23 (2019).
2. J. Broichhagen, J. A. Frank, D. Trauner, A Roadmap to Success in Photopharmacology. Acc. Chem. Res. 48, 1947-1960 (2015).
3. H. Bouas-Laurent, H. Dürr, Organic photochromism. Pure Appl. Chem. 73, 639-665 (2001).
4. M. Banghart, K. Borges, E. Isacoff, D. Trauner, R. H. Kramer, Light-activated ion channels for remote control of neuronal firing. Nat. Neurosci. 7, 1381-1386 (2004).
5. Z.-Y. Zhang, Y. He, Y. Zhou, C. Yu, L. Han, and T. Li, Pyrazolylazophenyl Ether-Based Photoswitches: Facile Synthesis, (Near-)Quantitative Photoconversion, Long Thermal Half-Life, Easy Functionalization, and Versatile Applications in Light-Responsive Systems, Chem. - A Eur. J. 25, 13402-13410 (2019).
6. Y. Kim, J. A. Phillips, H. Liu, H. Kang, W. Tan, Using photons to manipulate enzyme inhibition by an azobenzene-modified nucleic acid probe. Proc. Natl. Acad. Sci. U. S. A. 106, 6489-94 (2009).
7. R. J. Mart, R. K. Allemann, Azobenzene photocontrol of peptides and proteins. Chem. Commun. 52, 12262-12277 (2016).
8. W. Szymański, J. M. Beierle, H. A. V Kistemaker, W. A. Velema, B. L. Feringa, Reversible photocontrol of biological systems by the incorporation of molecular photoswitches. Chem. Rev. 113, 6114-6178 (2013).
9. A. S. Lubbe, W. Szymanski, B. L. Feringa, Recent developments in reversible photoregulation of oligonucleotide structure and function. Chem. Soc. Rev. 46, 1052-1079 (2017).
10. W. Szymański, D. Yilmaz, A. Koçer, B. L. Feringa, Bright ion channels and lipid bilayers. Acc. Chem. Res. 46, 2910-2923 (2013).
11. J. Levitz, J. Broichhagen, P. Leippe, D. Konrad, D. Trauner, E. Y. Isacoff, Dual optical control and mechanistic insights into photoswitchable group II and III metabotropic glutamate receptors. Proc. Natl. Acad. Sci. U. S. A. 114, E3546-E3554 (2017).
12. A. Hossion, M. Bio, G. Nkepang, S. G. Awuah, Y. You, Visible light controlled release of anticancer drug through double activation of prodrug. ACS Med. Chem. Lett. 4, 124-127 (2013).
13. J. Broichhagen, T. Podewin, H. Meyer-Berg, Y. Von Ohlen, N. R. Johnston, B. J. Jones, S. R. Bloom, G. A. Rutter, A. Hoffmann-Röder, D. J. Hodson, D. Trauner, Optical Control of Insulin Secretion Using an Incretin Switch. Angew. Chemie - Int. Ed. 54, 15565-15569 (2015).
14. W. Guo, A. E. Hight, J. X. Chen, N. C. Klapoetke, K. E. Hancock, B. G. Shinn-Cunningham, E. S. Boyden, D. J. Lee, D. B. Polley, Hearing the light: Neural and perceptual encoding of optogenetic stimulation in the central auditory pathway. Sci. Rep. 5 (2015).
15. I. Tochitsky, M. A. Kienzler, E. Isacoff, R. H. Kramer, Restoring Vision to the Blind with Chemical Photoswitches. Chem. Rev. 118, 10748-10773 (2018).
16. J. Font, M. López-Cano, S. Notartomaso, P. Scarselli, P. Di Pietro, R. Bresolí-Obach, G. Battaglia, F. Malhaire, X. Rovira, J. Catena, J. Giraldo, J.-P. Pin, V. Fernández-Dueñas, C. Goudet, S. Nonell, F. Nicoletti, A. Llebaria, F. Ciruela, Optical control of pain in vivo with a photoactive mGlu5 receptor negative allosteric modulator. Elife. 6 (2017).
17. C. E. E. Weston, A. Krämer, F. Colin, Ö. Yildiz, M. G. J. G. J. Baud, F.-J. Meyer-Almes, M. J. J. Fuchter, Toward Photopharmacological Antimicrobial Chemotherapy Using Photoswitchable Amidohydrolase Inhibitors. ACS Infect. Dis. 3, 152-161 (2017).
18. L. Stricker, M. Böckmann, T. M. Kirse, N. L. Doltsinis, B. J. Ravoo, Arylazopyrazole Photoswitches in Aqueous Solution: Substituent Effects, Photophysical Properties, and Host-Guest Chemistry. Chem. - A Eur. J. 24, 8639-8647 (2018).
19. J. D. Harris, M. J. Moran, I. Aprahamian, New molecular switch architectures. Proc. Natl. Acad. Sci. U. S. A. 115, 9414-9422 (2018).
20. H. V Patel, K. A. Vyas, S. P. Pandey, P. S. Fernandes, Reaction of 2, 3, 4-pentantrione-3-arylhydrazones with N, N-dimethylhydrazine: Formation of substituted 1H-pyrazoles via demethylation. Synth. Commun. 22, 3081-3087 (1992).
21. C. E. Weston, R. D. Richardson, P. R. Haycock, A. J. P. White, M. J. Fuchter, Arylazopyrazoles: Azoheteroarene photoswitches offering quantitative isomerization and long thermal half-lives. J. Am. Chem. Soc. 136, 11878-11881 (2014).
22. N. Möller, T. Hellwig, L. Stricker, S. Engel, C. Fallnich, B. J. Ravoo, Near-infrared photoswitching of cyclodextrin-guest complexes using lanthanide-doped LiYF4 upconversion nanoparticles. Chem. Commun. 53, 240-243 (2017).
23. J. Calbo, C. E. Weston, A. J. P. White, H. S. Rzepa, J. Contreras-García, M. J. Fuchter, Tuning azoheteroarene photoswitch performance through heteroaryl design. J. Am. Chem. Soc. 139, 1261-1274 (2017).
24. S. Ludwanowski, M. Ari, K. Parison, S. Kalthoum, P. Straub, N. Pompe, S. Weber, M. Walter, A. Walther, pH Tuning of Water-Soluble Arylazopyrazole Photoswitches. Chem. - A Eur. J. (2020).
25. D. T. Nguyen, M. Freitag, C. Gutheil, K. Sotthewes, B. J. Tyler, M. Böckmann, M. Das, F. Schlüter, N. L. Doltsinis, H. F. Arlinghaus, B. J. Ravoo, F. Glorius, An Arylazopyrazole-Based N-Heterocyclic Carbene as a Photoswitch on Gold Surfaces: Light-Switchable Wettability, Work Function, and Conductance. Angew. Chemie - Int. Ed. 59, 13651-13656 (2020).
26. Z.-Y. Zhang, Y. He, Y. Zhou, C. Yu, L. Han, T. Li, Pyrazolylazophenyl Ether-Based Photoswitches: Facile Synthesis, (Near-)Quantitative Photoconversion, Long Thermal Half-Life, Easy Functionalization, and Versatile Applications in Light-Responsive Systems. Chem. - A Eur. J. 25, 13402-13410 (2019).
27. R. Siewertsen, H. Neumann, B. Buchheim-Stehn, R. Herges, C. Näther, F. Renth, F. Temps, Highly efficient reversible Z-E photoisomerization of a bridged azobenzene with visible light through resolved S1(nπ*) absorption bands. J. Am. Chem. Soc. 131, 15594-15595 (2009).
28. A. Ansari, A. Ali, M. Asif, Shamsuzzaman, Review: biologically active pyrazole derivatives. New J. Chem. 41, 16-41 (2016).
29. F. K. Keter, J. Darkwa, Perspective: The potential of pyrazole-based compounds in medicine. BioMetals. 25, 9-21 (2012).
30. S. Kumari, S. Paliwal, R. Chauhan, Synthesis of pyrazole derivatives possessing anticancer activity: Current status. Synth. Commun. 44, 1521-1578 (2014).
31. L. Ding, L. Grehn, E. De Clercq, G. Andrei, R. Snoeck, J. Balzarini, B. Fransson, U. Ragnarsson, Synthesis and antiviral activity of three pyrazole analogues of distamycin A. Acta Chem. Scand. 48, 498-505 (1994).
32. A. Kalusalingam, I. Arumugam, R. Velayutham, U. Natarajan, A. J. S. Johnsamuel, P. Promwichit, Synthesis, characterization and anticonvulsant activity of some pyrazole derivatives. J. Glob. Pharma Technol. 3, 25-30 (2011).
33. M. Murahari, V. Mahajan, S. Neeladri, M. S. Kumar, Y. C. Mayur, Ligand based design and synthesis of pyrazole based derivatives as selective COX-2 inhibitors. Bioorg. Chem. 86, 583-597 (2019).
34. R. K. Malik, K. C. Reddy, in Proceedings of the 1999 Beltwide Cotton Conference, January 1999, Orlando, Florida, USA (1999), pp. 1293-1295.

## Claims

1. A compound of the formula:

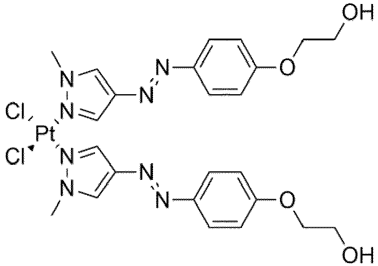

2. A method of producing the compound as defined in claim 1, **characterised in that** it comprises the following steps:
a) synthesis of a photoswitch is carried out, in which hydrochloric acid is added to an aqueous solution of 4-amino-1-methylpyrazole, and the mixture is cooled to 0-5°C, after which a pre-cooled aqueous solution of sodium nitrite is added while maintaining the cooling, and then an aqueous solution of phenol and sodium hydroxide at 0-5°C is added, then an aqueous solution of sodium carbonate at 0-5°C is added to the mixture and stirring is continued for at least 1 hour, maintaining the temperature at 0-5°C, then the pH of the mixture is adjusted to ~7 and the precipitate obtained is filtered and washed with water,
b) the precipitate obtained in step a) and potassium carbonate are dissolved in dimethylformamide, after which potassium iodide is added to the mixture, then 1-bromo-2-ethanol is added dropwise, and the reaction is carried out at a temperature of at least 110°C for at least 3 hours, after which the reaction is quenched by the addition of water and stirred until a precipitate crystallises, after which the precipitate is filtered and washed with water, dissolved in ethanol and dried with a drying agent, and then the precipitate obtained is subjected to purification with column chromatography,
(c) the purified precipitate from step b) which has previously been suspended in ethanol is added to an aqueous solution of potassium tetrachloroplatinate and the reaction is carried out for at least 2 hours, then a further portion of ethanol is added to the mixture and the mixture is stirred for at least 46 hours, then the ethanol is evaporated and the precipitate obtained is washed with diethyl ether and the precipitate is dried for at least 20 hours.

3. The compound of claim 1 or produced by the method of claim 2 for use in medicine, particularly in anti-cancer therapy.

## Patentansprüche

1. Verbindung der Formel:

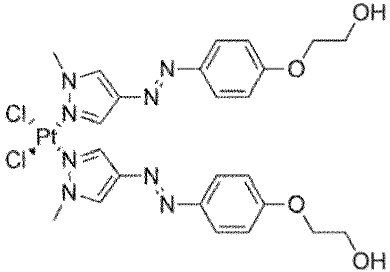

2. Verfahren zum Herstellen der in Fig. 1 definierten Verbindung, **gekennzeichnet durch** die folgenden Schritte:
a) es wird eine Synthese eines Photoschalters durchgeführt, in der Salzsäure zu einer wässrigen Lösung von 4-Amino-1-methylpyrazol hinzugefügt wird, und die Mischung wird auf 0-5°C abgekühlt, danach wird eine vorgekühlte wässrige Lösung von Natriumnitrit bei aufrechterhaltener Kühlung hinzugefügt, dann wird eine wässrige Lösung von Phenol und Natriumhydroxid bei 0-5°C hinzugefügt, dann wird eine wässrige Lösung von Natriumcarbonat bei 0-5°C zu der Mischung hinzugefügt und wird ein Rühren für wenigstens 1 Stunde fortgesetzt und die Temperatur bei 0-5°C gehalten, dann wird der pH-Wert der Mischung auf ~7 eingestellt und wird das Präzipitat gefiltert und mit Wasser gewaschen,
b) das in dem Schritt a) erhaltene Präzipitat und Kaliumcarbonat werden in Dimethylformamid gelöst, danach wird Kaliumiodid zu der Mischung hinzugefügt, dann wird 1-Brom-2-ethanol tropfenweise hinzugefügt, und die Reaktion wird bei einer Temperatur von wenigstens 110°C für wenigstens 3 Stunden durchgeführt, danach wird die Reaktion durch das Hinzufügen von Wasser gelöscht und gerührt, bis ein Präzipitat kristallisiert, dann wird das Präzipitat gefiltert und mit Wasser gewaschen, in Ethanol gelöst und mit einem Trocknungsmittel getrocknet, dann wird das Präzipitat einer Reinigung durch eine Säulenchromatographie unterworfen,
c) das gereinigte Präzipitat aus dem Schritt b), das zuvor in Ethanol suspendiert wurde, wird zu einer wässrigen Lösung aus Kaliumtetrachloridoplatinat hinzugefügt und die Reaktion wird für wenigstens 2 Stunden durchgeführt, dann wird eine weitere Menge von Ethanol zu der Mischung hinzugefügt und die Mischung wird für wenigstens 46 Stunden gerührt, dann wird das Ethanol verdampft und wird das erhaltene Präzipitat mit Diethylether gewaschen und wird das Präzipitat für wenigstens 20 Stunden getrocknet.

3. Verbindung, die gemäß Anspruch 1 beschaffen ist oder gemäß dem Verfahren von Anspruch 2 hergestellt ist und in der Medizin und insbesondere der Krebstherapie verwendet wird.

## Revendications

1. Composé de formule :

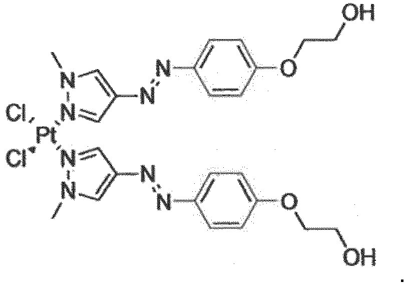

2. Procédé de production du composé tel que défini dans la revendication 1, **caractérisé en ce qu'**il comprend les étapes suivantes :
a) une synthèse d'un interrupteur photoélectrique est réalisée, dans laquelle de l'acide chlorhydrique est ajouté à une solution aqueuse de 4-amino-1-méthylpyrazole, et le mélange est refroidi à 0 à 5 °C, après quoi une solution aqueuse prérefroidie de nitrite de sodium est ajoutée tout en maintenant le refroidissement, puis une solution aqueuse de phénol et d'hydroxyde de sodium à 0 à 5 °C est ajoutée, puis une solution aqueuse de carbonate de sodium à 0 à 5 °C est ajoutée au mélange et l'agitation est poursuivie pendant au moins 1 heure en maintenant la température à 0 à 5 °C, puis le pH du mélange est ajusté à -7 et le précipité obtenu est filtré et lavé à l'eau,
b) le précipité obtenu à l'étape a) et du carbonate de potassium sont dissous dans du diméthylformamide, après quoi de l'iodure de potassium est ajouté au mélange, puis du 1-bromo-2-éthanol est ajouté goutte à goutte, et la réaction est effectuée à une température d'au moins 110 °C pendant au moins 3 heures, après quoi la réaction est trempée par l'addition d'eau et agitée jusqu'à ce qu'un précipité se cristallise, après quoi le précipité est filtré et lavé à l'eau, dissous dans de l'éthanol et séché à l'aide d'un agent de séchage, puis le précipité obtenu est soumis à une purification par chromatographie sur colonne,
c) le précipité purifié issu de l'étape b) qui a été préalablement mis en suspension dans de l'éthanol est ajouté à une solution aqueuse de tétrachloroplatinate de potassium et la réaction est effectuée pendant au moins 2 heures, puis une autre portion d'éthanol est ajoutée au mélange et le mélange est agité pendant au moins 46 heures, puis l'éthanol est évaporé et le précipité obtenu est lavé avec du diéthyléther et le précipité est séché pendant au moins 20 heures.

3. Composé selon la revendication 1 ou produit par le procédé selon la revendication 2, destiné à une utilisation en médecine, en particulier dans une thérapie anticancéreuse.
